(19) Europäisches Patentamt
European Patent Office
Office européen des brevets

(11) Veröffentlichungsnummer: **0 014 927**
**B1**

# (12) EUROPÄISCHE PATENTSCHRIFT

(45) Veröffentlichungstag der Patentschrift: **16.09.81**

(51) Int. Cl.³: **C 07 C 87/50,** C 07 C 85/145, C 07 C 85/24

(21) Anmeldenummer: **80100668.5**

(22) Anmeldetag: **11.02.80**

(54) **Verfahren zur Herstellung von Gemischen von Polyaminen der Polyamino-polyaryl-polymethylenreihe.**

(30) Priorität: **21.02.79 US 13765**

(43) Veröffentlichungstag der Anmeldung: **03.09.80 Patentblatt 80/18**

(45) Bekanntmachung des Hinweises auf die Patenterteilung: **16.09.81 Patentblatt 81/37**

(84) Benannte Vertragsstaaten: **BE DE FR GB IT NL**

(56) Entgegenhaltungen:
**EP-A1-0 003 303**
**DE-A1-2 748 968**
**GB-A-1 167 950**
**GB-A-1 231 980**
**US-A-3 857 890**
**US-A-4 071 558**

(73) Patentinhaber: **BAYER AG, Zentralbereich Patente, Marken und Lizenzen, D-5090 Leverkusen 1, Bayerwerk (DE)**
Patentinhaber: **Mobay Chemical Corporation, Penn Lincoln Parkway West, Pittsburgh, Pennsylvania 15205 (US)**

(72) Erfinder: **Dunlap, Kenneth L., 20 Rosary Road, New Martinsville West Virginia 26155 (US)**
Erfinder: **Knöfel, Hartmut, Dr., Duelmener Weg 21, D-5068 Odenthal (DE)**

(74) Vertreter: **Müller, Heinz-Gerd, Dipl.-Ing., c/o Bayer AG Zentralbereich Patente Marken und Lizenzen, D-5090 Leverkusen 1, Bayerwerk (DE)**

**EP 0 014 927 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Gemischen von Polyaminen der Polyamino-polyaryl-polymethylenreihe

Die Herstellung von Diaminoarylmethanen und Gemischen von Methylenbrücken aufweisenden Polyarylpolyaminen, die die Diaminoarylmethane enthalten, durch saure Kondensation von aromatischen Aminen und Formaldehyd ist bekannt (siehe beispielsweise US-PS 2 683 730). Die Diaminoarylmethane und die Polyamingemische sind wertvoll als Zwischenprodukte bei der Herstellung der entsprechenden Di- und Polyisocyanate.

Die Diaminoarylmethane, die nach den allgemein in der Technik angewendeten Verfahren hergestellt werden, bestehen hauptsächlich (d.h. zu 90 Gew.-% oder mehr) aus dem 4,4'-Isomeren und verhältnismässig geringen Mengen (d.h. weniger als 10 Gew.-%) des entsprechenden 2,4'-Isomeren. Vor kurzem zeigte sich Interesse an Diaminoarylmethanen, die grössere Mengen des 2,4'-Isomeren enthalten. Verschiedene Verfahren zur Herstellung dieser Gemische mit hohem Anteil des 2,4'-Isomeren wurden vorgeschlagen. Als Beispiele hierfür seien die Verfahren genannt, die in den US-PSen 3 362 979 (Verfahren unter Verwendung eines sauren, Siliciumdioxid enthaltenden Katalysators), 3 277 173 (in Gegenwart geregelter Wassermengen durchgeführtes Verfahren), 3 857 890 (Entfernung von Säure nach Bildung von Aminobenzylamin) und 4 071 558 (Verfahren unter Verwendung eines bestimmten Katalysators) und in der BE-PS 648 787 (Verfahren unter Verwendung geregelter Temperaturen) beschrieben werden. Beim Verfahren der GB-PS 1 167 950 erzielt man zwar einen relativ hohen Anteil an 2,4'-Isomeren im Zweikern-Anteil der Anilin/-Formaldehyd-Kondensate, jedoch muss dieser Vorteil mit den Nachteilen erkauft werden, dass die Umsetzung wasserfrei durchgeführt werden muss, d.h. dass teilweise feste Ausgangsmaterialien (Anilinsalz bzw. Paraformaldehyd) eingesetzt werden müssen, was in der grosstechnischen Praxis sowohl zu Schwierigkeiten bezüglich der Dosierung der Ausgangsmaterialien als auch bezüglich der Handhabbarkeit der in Abwesenheit hoher Anilinüberschüsse oftmals schlecht fliessfähigen Reaktionsgemische führt, und insbesondere, dass die relativ hohen Ausbeuten an 2,4'-Isomeren mit der Bildung von vergleichsweise hohen Mengen an Polyaminen mit mehr als 2 aminosubstituierten Benzolringen einhergeht. Auch das Verfahren der GB-PS 1 231 980 gestattet die Herstellung von Polyamingemischen der Diphenylmethanreihe mit einem hohen Gehalt an 2,4'-Isomeren im Zweikern-Anteil. Dieses Verfahren, welches bei sehr hohen Temperaturen unter Verwendung geringer Mengen an Säurekatalysator durchgeführt wird, führt jedoch wegen der hohen Reaktionstemperaturen zu unerwünschten Nebenprodukten und insbesondere zu hohen Konzentrationen an stets unerwünschten 2,2'-Isomeren im Reaktionsgemisch. Darüberhinaus muss auch beim Verfahren dieser Vorveröffentlichung die Bildung hoher Mengen von höherfunktionellen Polyaminen in Kauf genommen werden.

Das nachstehend näherbeschriebene erfindungsgemässe Verfahren eröffnet nun einen Weg zur Herstellung von Polyamingemischen der Diphenylmethanreihe mit einem hohen Gehalt an 2,4'-Isomeren, ohne mit den Nachteilen der gleichzeitigen Bildung von hohen Mengen an 2,2'-Isomeren und hohen Mengen an höherfunktionellen Polyaminen und der Notwendigkeit der ausschliesslichen Verwendung wasserfreier Ausgangsmaterialien behaftet zu sein.

Das nachstehend näherbeschriebene erfindungsgemässe Verfahren unterscheidet sich von dem Verfahren der nicht vorveröffentlichten, älteren EP-A 1-0003303 beispielsweise durch den Umstand, dass beim erfindungsgemässen Verfahren zunächst bei 10 bis 100 °C vorzugsweise unter Verwendung von lediglich einem Teil des insgesamt zum Einsatz gelangenden Säurekatalysators ein erstes Umlagerungsprodukt (N-(Aminobenzyl)-anilin) aus dem sich zunächst bildenden Vorkondensat (N,N'-Methylen-bis-anilin) gebildet wird, welches anschliessend vorzugsweise unter Zusatz einer weiteren Säuremenge bei 75–150 °C einer weiteren Umsetzung unterworfen wird, während gemäss älterer europäischer Patentanmeldung diese erfindungswesentliche Zwischenstufe übersprungen wird, da das zuletzt genannte Vorkondensat sofort mit der Gesamtmenge des Säurekatalysators und einer weiteren Menge Anilin bei 100–200 °C zum Endprodukt umgesetzt wird. Ein weiterer Unterschied liegt in dem Umstand begründet, was die Gesamtmenge des Säurekatalysators beim erfindungsgemässen Verfahren einem Protonierungsgrad von 10–100% beim Verfahren der älteren Anmeldung, jedoch einem Protonierungsgrad von 0,1–10% entspricht.

Die Erfindung wird nachstehend unter Bezugnahme auf die Abbildung beschrieben, die ein Fliessschema einer ausführlicher in Beispiel 6 beschriebenen Ausführungsform der Erfindung darstellt.

Die Erfindung ist auf die Herstellung von Polyaminen der Polyamino-polyaryl-polymethylen-Reihe nach einem Verfahren gerichtet, das dadurch gekennzeichnet ist, dass man

a) ein aromatisches Amin mit Formaldehyd in Gegenwart eines wässrigen sauren Katalysators bei einer Temperatur von 10 bis 100 °C umsetzt oder ein Kondensat, das aus einem aromatischen Amin und Formaldehyd in Abwesenheit eines sauren Katalysators erhalten worden ist, einer ersten Umlagerung in Gegenwart eines wässrigen sauren Katalysators bei einer Temperatur von 10 ° bis 100 °C unterwirft und hierdurch ein sekundäres Amin enthaltendes Zwischenproduktgemisch bildet, wobei die verwendete Säuremenge einem Protonierungsgrad von 0,1 bis 25% entspricht,

b) wässrige Säure als Katalysator dem in der Stufe (a) erhaltenen Gemisch in einer solchen Menge

zusetzt, dass die gesamte Säuremenge im Gemisch einem Protonierungsgrad von 10 bis 100% entspricht,

c) das in der Stufe (b) enthaltene Gemisch einer Umlagerungsreaktion bei einer Temperatur von 75 ° bis 150 °C unterwirft und hierdurch ein Kondensationsgemisch bildet, das das Gemisch von Polyaminen enthält, und

d) die Polyamine isoliert.

Der hier gebrauchte Ausdruck «Protonierungsgrad» wird definiert als Prozentsatz aller Aminstickstoffatome, die protoniert worden sind, d.h. die in Form von Ammoniumsalzen vorliegen.

Es ist allgemein bekannt, dass die saure Kondensation von aromatischen Aminen und Formaldehyd in zwei getrennten Stufen stattfindet. Um dies zu veranschaulichen, läuft beispielsweise im Falle von Anilin und Formaldehyd die Reaktionsfolge wie folgt ab:

(A) 2 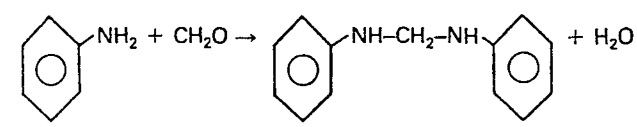

I (Vorkondensation)

(B) I + 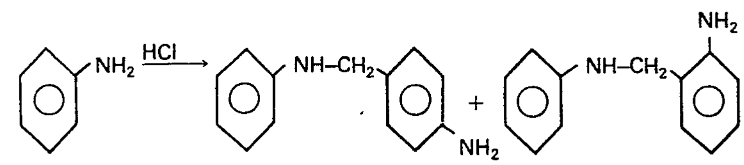


III (Umlagerung I) IV

(C) III + 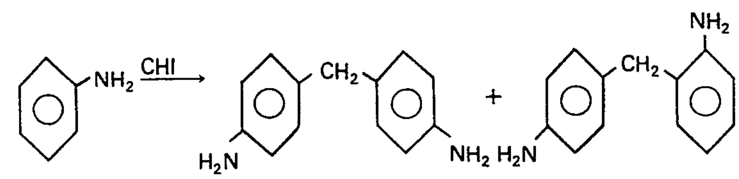


V (Umlagerung II) VI

(D) IV + 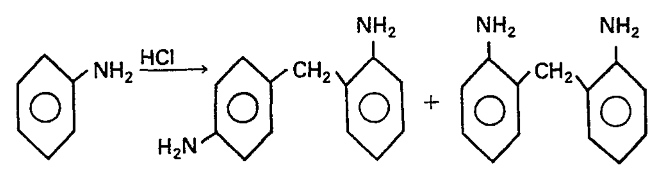


Bekanntlich führt die Umlagerung von N-(p-Aminobenzyl)-anilin III nur zur Bildung der 2,4'- und 4,4'-Isomeren (d.h. Sequenz (C)), während die Umlagerung von N-(o-Aminobenzyl)anilin IV nur zur Bildung der 2,2'- und 2,4'-Isomeren führt.

Es ist bekannt, dass die Bildung der N-(Aminobenzyl)amine III und IV von der Bildung der entsprechenden oligomeren Produkte begleitet ist. Das Gemisch von Isomeren des Typs III und IV und ihrer oligomeren Formen wird nachstehend als «sekundäres Amin enthaltendes Zwischenproduktgemisch» bezeichnet.

Bei der vorstehend dargestellten endgültigen Umlagerungsreaktion erfolgt eine Umlagerung der Zwischenprodukte III und IV unter Bildung der entsprechenden Diaminophenylmethane. Das 2,2'-Isomere wird ebenfalls, wenn auch in verhältnismässig geringer Menge, bei weitgehend katalysierten üblichen Verfahren gebildet. Es ist ferner bekannt, dass die endgültige Umlagerung auch die entsprechenden Polyamine ergibt.

Von den vorstehend genannten Stufen finden die ersten beiden, d.h. die Vorkondensation (die auch in Abwesenheit eines sauren Katalysators stattfindet und daher auch in einer ersten unabhängigen Stufe, d.h. bei 10 ° bis 100 °C in Abwesenheit eines Katalysators durchgeführt werden kann) und die erste Umlagerung, finden im allgemeinen statt, wenn das Anilin und der Formaldehyd in Gegenwart des sauren Katalysators bei Temperaturen von 10 ° bis 100 °C zusammengeführt werden. Die Reaktion ist exotherm, und die Temperatur steigt erheblich, wenn sie nicht durch Kühlen geregelt wird. Die Endstufe des Verfahrens, d.h. die Umlagerung der Zwischenprodukte, findet in wesentlichem Ausmass erst statt, wenn das Reaktionsgemisch – im allgemeinen auf eine Temperatur über 60 °C – erhitzt wird.

Das Ziel der Erfindung besteht im wesentlichen darin, die gebildete Menge des Zwischenprodukts des Typs IV zu steigern und hierdurch das Ausmass der o-Substitution im Endprodukt zu steigern. Es wurde nun gefunden, dass das Ausmass der o-Substitution überraschend gesteigert wird, wenn der saure Katalysator in zwei oder mehr Stufen zugesetzt wird. Die erste Zugabe erfolgt zu

Beginn der Reaktion. Dies bedeutet, dass der erste Teil des Katalysators entweder dem Kondensat, dass aus dem aromatischen Amin und dem Formaldehyd in Abwesenheit von Katalysatoren erhalten worden ist, oder dem Reaktionsgemisch, das aromatisches Amin und Formaldehyd enthält, zugesetzt wird, während die nächste Zugabe oder die nächsten Zugaben nach der Bildung des Zwischenproduktgemisches, jedoch vor Vollendung der endgültigen Umlagerung erfolgen.

Bei der Durchführung des Verfahrens gemäss der Erfindung erfolgt die Bildung des sekundäres Amin enthaltenden Zwischenproduktgemisches in üblicher Weise mit Ausnahme der zu Beginn vorhandenen Säuremenge. Hierauf wird nachstehend eingegangen. Im allgemeinen werden entweder das aromatische Amin und der Formaldehyd unter wässrigen Bedingungen in Gegenwart der vorgeschriebenen Katalysatormenge zusammengebracht, oder die vorgeschriebene Katalysatormenge wird dem aus dem aromatischen Amin und dem Formaldehyd gebildeten Kondensat in völliger Abwesenheit eines Katalysators zugesetzt. Die Reihenfolge, in der die Reaktionsteilnehmer zusammengeführt werden, ist nicht entscheidend wichtig, jedoch erweist es sich als vorteilhaft, den Formaldehyd einem vorgebildeten Gemisch von aromatischem Amin und Säure zuzusetzen.

Die Kondensationsreaktion zwischen dem aromatischen Amin und dem Formaldehyd ist exotherm. Gegebenenfalls kann das Reaktionsgemisch gekühlt werden, um zu verhindern, dass die Reaktionstemperatur über eine gewünschte Höhe steigt. Die Reaktionstemperatur kann auch durch die Geschwindigkeit, mit der die Reaktionsteilnehmer zusammengeführt werden, geregelt werden. Die Reaktion gemäß der Stufe (a) findet bei einer Temperatur im Bereich von 10–100 °C statt. Die Reaktionstemperatur wird vorzugsweise im Bereich von etwa 40 °C bis 100 °C gehalten. Man kann die Reaktionstemperatur bei einem bestimmten Wert in diesem Bereich halten oder sie nach Belieben steigen oder fallen lassen, indem man die Geschwindigkeiten der Zugabe der Reaktionsteilnehmer oder andere Faktoren regelt, vorausgesetzt, dass die Reaktionstemperatur den Bereich innerhalb der vorstehend genannten Grenzen nicht verlässt. Es hat sich gezeigt, dass die höheren Temperaturen im vorstehend genannten Bereich leichter in Kauf genommen werden können, wenn die Menge der ursprünglich im Reaktionsgemisch vorhandenen Säure verringert wird.

Das Mengenverhältnis, in dem das aromatische Amin, der Formaldehyd und der vorgelegte saure Katalysator zusammengeführt werden, bestimmt die Gesamtausbeute an Diamin und in einem gewissen Ausmass den Anteil von o- und p-Substitution im Endprodukt. Vorteilhaft beträgt das Molverhältnis von aromatischem Amin zu Formaldehyd 2:1 bis 15:1, vorzugsweise 3:1 bis 10:1. Während die untere Grenze der Konzentration des aromatischen Amins zu Formaldehyd entscheidend für das beim Verfahren erreichte Gesamtergebnis ist, besteht für die obere Grenze des Mengenverhältnisses diese entscheidende Bedeutung nicht; vielmehr wird sie weitgehend durch wirtschaftliche Erwägungen diktiert.

Die in der ersten Stufe des Verfahrens gemäss der Erfindung verwendete Menge des sauren Katalysators kann in weiten Grenzen liegen und entspricht einem Protonierungsgrad von 0,1 bis 25%. Vorzugsweise entspricht die zu Beginn verwendete Säuremenge einem Protonierungsgrad von 0,2 bis 10%, wobei ein Protonierungsgrad von 0,3 bis 7% besonders bevorzugt wird.

Alle bisher im Stand der Technik verwendeten sauren Katalysatoren einschliesslich der in den vorstehend genannten Veröffentlichungen speziell genannten Katalysatoren können beim Verfahren gemäss der Erfindung als Katalysatoren verwendet werden. Geeignet als Katalysatoren sind beispielsweise wasserlösliche Säuren mit pKa-Werten unter 2,5, vorzugsweise unter 1,5. Spezielle Beispiele sind Salzsäure, Bromwasserstoffsäure, Schwefelsäure, Trifluoressigsäure, Methansulfonsäure, Trifluormethansulfonsäure, Benzolsulfonsäure und Phosphorsäure. Salzsäure wird als Katalysator bevorzugt. Die vorstehend genannten Säuren können auch in Mischung mit sauren oder neutralen Salzen solcher Säuren, beispielsweise den entsprechenden Ammoniumsalzen oder den entsprechenden Alkalisalzen, verwendet werden. Falls gewünscht, können die aus dem vorstehend genannten Säuren und aus dem als Ausgangsmaterial verwendeten aromatischen Amin gebildeten oder als Kondensationsprodukte in einer beliebigen Stufe des Verfahrens entstehenden Ammoniumsalze als einzige Katalysatoren verwendet werden.

Der Fortschritt der Kondensation des aromatischen Amins und des Formaldehyds in der vorstehend genannten ersten Reaktionsstufe (a) kann leicht mit Hilfe üblicher Analysenmethoden, z.B. Infrarotspektroskopie, NMR-Spektroskopie u.dgl., verfolgt werden. Die Bildung des das sekundäre Amin enthaltenden Zwischenproduktgemisches wird festgestellt und nach einer beliebigen Methode dieser Art verfolgt. Im allgemeinen kann die zusätzliche Menge des Säurekatalysators zu einer beliebigen Zeit zugegeben werden, nachdem die Zwischenprodukte nachgewiesen worden sind. Die zusätzliche Säure, die in allen Fällen zugegeben werden muss, kann somit zu jeder Zeit nach der Feststellung des Zwischenproduktgemisches und vor der Vollendung der endgültigen Umlagerungsstufe der Kondensation zugesetzt werden. Die Menge des zugegebenen zusätzlichen Säurekatalysators wird so bemessen, dass die Gesamtmenge der Säure im Gemisch (einschliesslich der zu Beginn zugesetzten Säure) einem Protonierungsgrad von 10 bis 100%, vorzugsweise von 20 bis 50% entspricht.

Die Zeit, während der das Gemisch in der Endstufe erhitzt wird, hängt von den in jedem gegebenen Fall angewendeten Reaktionsbedingungen, insbesondere von der Reaktionstemperatur ab. Die Temperatur in der Endstufe liegt im Bereich von 75 ° bis 150 °C. Der Fortschritt der Reaktion kann nach üblichen Anlysenmethoden wie Infrarotspektroskopie, Gas-Flüssigkeitschromatographie u.dgl. verfolgt werden. Wenn die Reaktion nach einer dieser Analysenmethoden als vollendet festgestellt wird, wird das Reaktionsgemisch in beliebiger geeigneter Weise in Abhängigkeit vom vorgesehenen Verwendungszweck des erhaltenen Gemisches von Polyaminen aufgearbeitet. Beispielsweise kann das Reaktionsgemisch neutralisiert und zur Entfernung von überschüssigem aromatischem Amin destilliert werden, und das hierbei erhaltene Gemisch von Polyaminen wird als solches isoliert. Es ist auch möglich, das letztgenannte Gemisch weiter zu destillieren oder einer Trennung auf anderem Wege, z.B. durch Chromatographie, fraktionierte Kristallisation u.dgl., zu unterwerfen, um die im Reaktionsgemisch vorhandenen gemischten oder nicht gemischten Diaminoarylmethane teilweise oder ganz zurückzugewinnen.

Eine der günstigsten Methoden zur Isolierung der Endprodukte aus dem Reaktionsgemisch besteht darin, sie aus der wässrigen Lösung mit Hilfe eines Gemisches eines hydrophoben Lösungsmittels mit dem als Ausgangsmaterial verwendeten aromatischen Amin zu extrahieren, wie in der US-PS 3 996 283 beschrieben. Bei Anwendung des Extraktionsverfahrens gemäss der Erfindung wird die wässrige Phase zur ersten und zweiten Stufe in den Mengen, die den vorstehend genannten Protonierungsgraden entsprechen, im Kreislauf zurückgeführt.

Wenn mit hohen Anilin/Formaldehyd-Verhältnissen und/oder hohen Protonierungsgraden in der Stufe (b) gearbeitet wird, wird im allgemeinen das Extraktionsverfahren zur Produktgewinnung angewendet. Umgekehrt wird bei niedrigem Anilingehalt und niedrigen Protonierungsgraden vorzugsweise die Neutralisationstechnik für die Produktgewinnung angewendet.

Das direkt aus der vorstehend beschriebenen Reaktion erhaltene Gemisch von Polyaminen oder das Gemisch von Polyaminen, das nach teilweiser oder vollständiger Entfernung der darin enthaltenen Diaminoarylmethane verbleibt, kann für alle beliebigen Zwecke, für die solche Gemische im Stand der Technik verwendet werden, eingesetzt werden. Beispielsweise kann das Gemisch der Polyamine als Zwischenprodukt für die Herstellung der entsprechenden Polymethylenpolyarylpolyisocyanate durch Phosgenierung verwendet werden. Die letzteren werden allgemein für die Herstellung von Polyurethan-Schaumstoffen verwendet.

Die Diaminoarylmethane, die in der vorstehend beschriebenen Weise isoliert werden können, eignen sich auch für alle Zwecke, für die solche Gemische üblicherweise verwendet werden. Beispielsweise können sie als Härtemittel für Epoxyharze oder als Zwischenprodukte für die Herstellung der entsprechenden Diisocyanatoarylmethane durch Phosgenierung verwendet werden.

Das Verfahren gemäss der Erfindung kann mit beliebigen aromatischen Aminen, z.B. Anilin, o-, m- oder p-Chloranilin, o-, m- oder p-Bromanilin, o-, m- oder p-Anisidin, o-, m- oder p-Phenetidin, o-, m- oder p-Toluidin, o-, m- oder p-Äthylanilin, o-, m- oder p-Isopropylanilin, o-, m- oder p-Xylidinen, α- oder β-Naphthylamin, o-, m- oder p-Benzylanilin, o-, m- oder p-Cyclohexylanilin, 2,6-Dimethylanilin, 2,6-Diäthylanilin, 2,6-Diisopropylanilin, 2,4- oder 2,6-Diaminotoluol, o-, m- oder p-Diaminobenzol, N-Methyl-, N-Äthyl-, N-Propyl-, N-Butyl-, N-Hydroxyäthyl- oder N-Chloräthylanilin, o-, m- oder p-Methyl-N-methylanilin, o-, m- oder p-Methyl-N-äthylanilin, o-, m- oder p-Chlor-N-methylanilin, o-, m- oder p-Chlor-N-äthylanilin, o-, m- oder p-Thioanisidin oder beliebigen Gemischen der vorstehend genannten Amine oder Gemischen der vorstehend genannten Amine mit ihren Formaldehyd-Kondensationsprodukten vom Diarylmethantyp durchgeführt werden. Vorzugsweise wird Anilin als Ausgangsamin beim Verfahren gemäss der Erfindung verwendet.

Der beim Verfahren gemäss der Erfindung verwendete Formaldehyd kann in Form einer wässrigen oder wässrig-methanolischen Lösung oder auch in Form eines Formaldehyd-Donators, z.B. Methylal, Trioxan oder Paraformaldehyd, eingesetzt werden. Vorzugsweise wird eine wässrige Formaldehydlösung beim Verfahren gemäss der Erfindung verwendet.

Wie bereits erwähnt, enthalten die beim Verfahren gemäss der Erfindung erhaltenen Gemische, ob in Form des isolierten Diamins oder der Gemische von Methylenbrücken aufweisenden Polyarylaminen, einen wesentlichen Anteil der o-Substitution. Die Gemische gemäss der Erfindung enthalten im allgemeinen 70 bis 95 Gew.-% des Diamins, während der Rest aus höheren Aminen besteht. Im Falle von Anilin/Formaldehyd-Produkten enthalten die Gemische im allgemeinen nicht mehr als 5 Gew.-% (bezogen auf vorhandenes Diamin) des 2,2'-Isomeren, wobei der Gehalt an 2,4'-Isomeren bis zu 40 Gew.-% beträgt.

Das Verfahren gemäss der Erfindung kann chargenweise oder teilweise oder vollständig kontinuierlich durchgeführt werden. Beispielsweise kann die erste Stufe im Reaktionsprozess (d.h. die Kondensation des aromatischen Amins und des Formaldehyds) in einzelnen Chargen oder in einem kontinuierlichen Prozess, bei dem die Reaktionsteilnehmer kontinuierlich im geeigneten Mengenverhältnis einem Ende einer Reaktionszone zugeführt werden und das Gemisch der Aminoarylamine kontinuierlich vom anderen Ende der Reaktionszone abgezogen wird, durchgeführt werden. Der zusätzliche Säurekatalysator kann dem erhaltenen Gemisch chargenweise oder kontinuierlich zugesetzt werden. Das Gemisch kann dann kontinuierlich durch eine Reaktionszone geführt werden, die bei einer Temperatur in dem Bereich, der zur Vollendung der ersten Stufe des Verfahrens erforderlich ist, gehalten wird. Das gewünschte Endprodukt wird vom anderen Ende der Reaktionszone kontinuierlich abgezogen. Die Durchlaufmenge wird so eingestellt, dass die Verweilzeit des Gemisches ausreicht, um die Umwandlung der Aminoarylamine in das gewünschte Gemisch der Methylenbrücken aufweisenden Polyarylamine zu erreichen.

Die Erfindung wird durch die folgenden Beispiele weiter erläutert. Diese Beispiele beschreiben die zur Zeit beste Ausführungsform der Erfindung.

Beispiel 1 (Vergleichsbeispiel)

Bei 40 °C wurden 100 g 30%iger wässriger Formaldehyd unter Rühren zu einem Gemisch von 372 g frisch destilliertem Anilin und 162 g 30%iger Salzsäure gegeben (0,3375 Mol Säure pro Mol Anilin, Protonierungsgrad 33,75%). Das Gemisch wurde 1 Stunde bei 40 °C gehalten. Es wurde dann auf die Rückflusstemperatur gebracht und 3 Stunden am Rückfluss erhitzt. Das Gemisch wurde mit überschüssigem Natriumhydroxid neutralisiert, von der wässrigen Solelösung abgetrennt und von restlichem Wasser und überschüssigem Anilin befreit. Das erhaltene Polyamin bestand aus 81,9% Diaminodiphenylmethan und 18,1% höheren Oligomeren. Das Diaminodiphenylmethan hatte die folgende Zusammensetzung: 0,3% 2,2'-Isomeres, 9,3% 2,4'-Isomeres und 90,3% 4,4'-Isomeres.

Beispiel 2

Bei 40 °C wurden 100 g 30%iger wässriger Formaldehyd unter Rühren zu einem Gemisch von 372 g Anilin und 8 g 30%iger Salzsäure gegeben (0,017 Mol Säure pro Mol Anilin; Protonierungsgrad 1,7%. Die Temperatur wurde 1 Stunde bei 40 °C gehalten und dann für 1 Stunde auf 100 °C erhöht. Nach Zugabe von 154 g 30%iger Salzsäure zum Gemisch (0,35 Mol Gesamtsäure pro Mol Anilin; Protonierungsgrad 35%) wurde das Reaktionsgemisch weitere 3 Stunden am Rückfluss erhitzt. Das Gemisch wurde neutralisiert, von der wässrigen Sole abgetrennt und von restlichem Wasser und überschüssigem Anilin befreit. Das erhaltene Polyamin enthielt 77,2% Diaminodiphenylmethan und 22,8% höhere Oligomere. Das Diamin bestand aus 0,9% 2,2'-Isomerem, 16,2% 2,4'-Isomerem und 82,9% 4,4'-Isomerem.

Beispiel 3

Bei 100 °C wurden 100 g einer wässrigen Formaldehydlösung unter Rühren zu einem Gemisch von 558 g Anilin und 3,7 g 30%iger Salzsäure gegeben (0,005 Mol Säure pro Mol Anilin; Protonierungsgrad 0,5%). Das Gemisch wurde 30 Minuten am Rückfluss erhitzt. Nach Zugabe von 158,5 g 30%iger Salzsäure zum Gemisch (0,23 Mol Gesamtsäure pro Mol Anilin; Protonierungsgrad 23%) wurde das Reaktionsgemisch weitere 3 Stunden am Rückfluss erhitzt. Nach Neutralisation und normaler Aufarbeitung (wie in den vorstehenden Beispielen beschrieben) enthielt das Polyamin 84,4% Diaminodiphenylmethan und 15,6% höhere Oligomere. Das Diamin bestand aus 2,5% 2,2'-Isomerem, 25,1% 2,4'-Isomerem und 71,3% 4,4'-Isomerem.

Beispiel 4

Bei 100 °C wurden 50 g wässrige 30%ige Formaldehydlösung unter Rühren zu einem Gemisch von 465 g Anilin und 10,1 g 30%iger Salzsäure gegeben (0,017 Mol Säure pro Mol Anilin; Protonierungsgrad 1,7%). Das Gemisch wurde 60 Minuten am Rückfluss erhitzt, worauf 125,1 g 30%ige Salzsäure zugesetzt wurden (0,22 Mol Gesamtsäure pro Mol Anilin; Protonierungsgrad 22%). Dieses Reaktionsgemisch wurde weitere 2,5 Stunden am Rückfluss erhitzt und dann mit Natriumhydroxid neutralisiert und normal aufgearbeitet (wie in Beispiel 1 beschrieben). Das Polyamin bestand aus 89,0% Diaminodiphenylmethan und 11,0% höheren Oligomeren. Das Diamin hatte die folgende Isomerenverteilung: 3,2% 2,2'-Isomeres, 30,6% 2,4'-Isomeres und 66,0% 4,4'-Isomeres.

Beispiel 5

Bei 80 °C wurden 100 g 30%ige wässrige Formaldehydlösung unter Rühren zu einem Gemisch von 558 g Anilin und 12 g 30%iger Salzsäure gegeben (0,017 Mol Säure pro Mol Anilin; Protonierungsgrad 1,7%). Das Reaktionsgemisch wurde 1 Stunde bei 80 °C gehalten, worauf die Temperatur auf 100 °C erhöht und 1 Stunde bei 100 °C gehalten wurde. Nach Zusatz von 150 g 30%iger HCl zum Gemisch (0,23 Mol Gesamtsäure pro Mol Anilin; Protonierungsgrad 23%) wurde das Gemisch weitere 3 Stunden am Rückfluss erhitzt. Nach Neutralisation und normaler Aufarbeitung (wie in Beispiel 1 beschrieben) wurde festgestellt, dass das Produkt aus 83,8% Diaminodiphenylmethan und 16,2% höheren Oligomeren bestand. Das Diamin bestand aus 2,5% 2,2'-Isomerem, 25,4% 2,4'-Isomerem und 72,1% 4,4'-Isomerem.

Beispiel 6

Dieses Beispiel beschreibt die Produktgewinnung durch Extraktion unter Bezugnahme auf die Abbildung.

Unter Verwendung einer kontinuierlich arbeitenden Laboratoriumsprüfapparatur werden im ersten Gefäss eines aus 6 Gefässen mit Rührer bestehenden Reaktors (3–8) die Ströme (A) (Anilin vom Vorratsbehälter (2)) und (B) (im Kreislauf geführte wässrige Anilin- oder Polyarylaminhydrochloridlösung vom Extraktor (10)) kontinuierlich gemischt und bei 80 °C mit wässriger Formalinlösung (Strom C) vom Gefäss (1) umgesetzt. Die Ströme haben die folgende Zusammensetzung (in g/Std.):

| | | | |
|---|---|---|---|
| Strom | (A) | 2050 | Anilin |
| | (B) | 171 | Anilin |
| | | 16 | Polyarylamin |
| | | 70 | Chlorwasserstoff |
| | | 315 | Wasser |
| | (C) | 120 | Formaldehyd |
| | | 280 | Wasser |

Nach einer mittleren Verweilzeit von etwa 25 Minuten wird eine weitere Menge der wässrigen Arylaminohydrochloridlösung, die vom Extraktor (10) im Kreislauf geführt wird; dem zweiten Gefäss (4) bei 95 °C zugesetzt (Strom D):

| | | | |
|---|---|---|---|
| Strom | (D) | 1370 | Anilin |
| | | 128 | Polyarylamin |
| | | 560 | Chlorwasserstoff |
| | | 2524 | Wasser |

Nach einer durchschnittlichen Verweilzeit von 10 Minuten im Gefäss (4) werden etwa 350 g/Std. Wasser (Strom (E) in einem weiteren Rührgefäss (5) unter Normaldruck abdestilliert und beispielsweise im Vorratsbehälter (13) gesammelt oder verworfen. Anschliessend wird die Reaktion innerhalb einer Stunde in den Gefässen (6) bis (8) unter Druck bei 120 bis 130 °C zu Ende geführt.

Die vollständig ausreagierte Lösung, die auf 95 °C gekühlt worden ist, wird anschliessend in ein Extraktionssystem eingeführt, das aus einer ersten und einer zweiten Extraktionskolonne besteht. In der ersten Extraktionskolonne (9) wird das Reaktionsprodukt bei etwa 95 °C aus der wässrigen Phase unter Verwendung der organischen Phase aus der zweiten Extraktionskolonne (10) extrahiert. (Das Gewichtsverhältnis von Anilin zu Xylol wird mit Anilin aus dem Behälter (2) auf 1,2:1,0 eingestellt.) Etwa 6500 g/Std. o-Xylol werden als Extraktionsmittel für die zweite Extraktionskolonne (10) verwendet und in der Destillationsstufe (11) zurückgewonnen.

Die in der Kolonne (10) erhaltene wässrige Arylaminohydrochloridlösung wird in die Ströme (B) und (D) unterteilt und im Kreislauf zurückgeführt.

Die aus dem Extraktionssystem austretende organische Phase wird im nachgeschalteten zweistufigen Destillationssystem in o-Xylol (erste Destillationsstufe (11)), Anilin (zweite Destillationsstufe (12)) und Polyarylamin (Destillationsrückstand von (12)) getrennt. Das zurückgewonnene Anilin wird zusammen mit dem dem System zugeführten raffinierten Anilin über das Gefäss (2) in die Reaktion eingesetzt.

Das Produkt, das bei dem beschriebenen Beispiel dieser Ausführungsform des Verfahrens gemäss der Erfindung gebildet worden ist (etwa 750 g/Std.), wird im Gefäss (14) aufgefangen und hat die folgende durchschnittliche Zusammensetzung:

88 Gew.-% Diaminodiphenylmethan
12 Gew.-% Polyamine mit hoher Ringzahl

Die Diaminofraktion hat die folgende durchschnittliche Zusammensetzung:

1,4 Gew.-% 2,2'-Diaminodiphenylmethan
18,5 Gew.-% 2,4'-Diaminodiphenylmethan
80,1 Gew.-% 4,4'-Diaminodiphenylmethan

## Patentansprüche

1. Verfahren zur Herstellung von Gemischen von Polyaminen der Polyamino-polyaryl-polymethylen-Reihe, dadurch gekennzeichnet, dass man
a) ein aromatisches Amin mit Formaldehyd in Gegenwart eines wässrigen sauren Katalysators bei einer Temperatur von 10 ° bis 100 °C umsetzt oder ein Kondensat, das aus einem aromatischen Amin und Formaldehyd in Abwesenheit eines sauren Katalysators erhalten worden ist, einer ersten Umlagerung in Gegenwart eines wässrigen sauren Katalysators bei einer Temperatur von 10 ° bis 100 °C unterwirft und hierdurch ein sekundäres Amin enthaltendes Zwischenproduktgemisch bildet, wobei die verwendete Säuremenge einem Protonierungsgrad von 0,1 bis 25% entspricht,
b) wässrige Säure als Katalysator dem in der Stufe (a) erhaltenen Gemisch in einer solchen Menge zusetzt, dass die gesamte Säuremenge im Gemisch einem Protonierungsgrad von 10 bis 100% entspricht,
c) das in der Stufe (b) erhaltene Gemisch einer Umlagerungsreaktion bei einer Temperatur von 75 ° bis 150 °C unterwirft und hierdurch ein die Polyamine enthaltendes saures Kondensationsgemisch bildet und
d) die Polyamine isoliert.

2. Verfahren nach Anspruch 1, dadurch gekennzeichnet, dass man als aromatisches Amin Anilin verwendet.

3. Verfahren nach Anspruch 1, dadurch gekennzeichnet, dass die in der Stufe (a) verwendete Säuremenge einem Protonierungsgrad von 0,2 bis 10% entspricht.

4. Verfahren nach Anspruch 3, dadurch gekennzeichnet, dass die in der Stufe (a) verwendete Säuremenge einem Protonierungsgrad von 0,3 bis 7% entspricht.

5. Verfahren nach Anspruch 3, dadurch gekennzeichnet, dass die Gesamtmenge der Säure in der Stufe (b) einem Protonierungsgrad von 20 bis 50% entspricht.

6. Verfahren nach Anspruch 1, dadurch gekennzeichnet, dass das Molverhältnis von aromatischem Amin zu Formaldehyd 2:1 bis 15:1 beträgt.

7. Verfahren nach Anspruch 6, dadurch gekennzeichnet, dass das Molverhältnis von aromatischem Amin zu Formaldehyd 3:1 bis 10:1 beträgt.

8. Verfahren nach Anspruch 1, dadurch gekennzeichnet, dass man als Säurekatalysator Salzsäure verwendet.

## Revendications

1. Procédé de préparation de mélanges de polyamines de la série des polyamino-polyaryl-polyméthylènes, caractérisé en ce que:
a) on fait réagir une amine aromatique avec du formaldéhyde en présence d'un catalyseur acide aqueux à une température de 10 à 100 °C ou on soumet un condensat obtenu à partir d'une amine aromatique et de formaldéhyde en absence d'un catalyseur acide, à une première transposition en présence d'un catalyseur acide aqueux à une température de 10 à 100 °C, formant ainsi un mélange de produits intermédiaires contenant une amine secondaire, la quantité d'acide utilisée correspondant à un degré de protonation de 0,1 à 25%,
b) au mélange obtenu lors de l'étape (a), comme catalyseur, on ajoute un acide aqueux en une quantité calculée de telle sorte que la quantité totale d'acide du mélange corresponde à un degré de protonation de 10 à 100%,
c) on soumet le mélange obtenu lors de l'étape (b) à une réaction de transposition à une température de 75 à 150 °C, formant ainsi un mélange de condensation acide contenant les polyamines, et
d) on isole les polyamines.

2. Procédé suivant la revendication 1, caractérisé en ce que, comme amine aromatique, on utilise l'aniline.

3. Procédé suivant la revendication 1, caractérisé en ce que la quantité d'acide utilisé lors de l'étape (a) correspond à un degré de protonation de 0,2 à 10%.

4. Procédé suivant la revendication 3, caractérisé en ce que la quantité d'acide utilisé lors de l'étape (a) correspond à un degré de protonation de 0,3 à 7%.

5. Procédé suivant la revendication 3, caractérisé en ce que la quantité totale d'acide utilisé lors de l'étape (b) correspond à un degré de protonation de 20 à 50%.

6. Procédé suivant la revendication 1, caractérisé en ce que le rapport molaire entre l'amine aromatique et le formaldéhyde est de 2:1 à 15:1.

7. Procédé suivant la revendication 6, caractérisé en ce que le rapport molaire entre l'amine aromatique et le formaldéhyde est de 3:1 à 10:1.

8. Procédé suivant la revendication 1, caractérisé en ce que, comme catalyseur acide, on utilise l'acide chlorhydrique.

**Claims**

1. Process for the production of mixtures of polyamines of the polyamino-polyaryl-polymethylene series, characterised in that

a) an aromatic amine is reacted with formaldehyde in the presence of an aqueous acid catalyst at a temperature of from 10° to 100 °C or a condensate which has been obtained from an aromatic amine and formaldehyde in the absence of an acid catalyst is subjected to a first rearrangement in the presence of an aqueous acid catalyst at a temperature of from 10° to 100 °C and a secondary amine-containing intermediate mixtures is thereby formed, the amount of acid employed corresponding to a protonation degree of from 0,1 to 25%,

b) aqueous acid is added as catalyst to the mixture obtained in step (a) in an amount such that the total amount of acid corresponds to a protonation degree of from 10 to 100%,

c) the mixture obtained in step (b) is subjected to a rearrangement reaction at a temperature of from 75° to 150 °C and an acidic condensation mixture containing the polyamines is thereby formed and

d) the polyamines are recovered.

2. Process according to claim 1, characterised in that the aromatic amine used is aniline.

3. Process according to claim 1, characterised in that the amount of acid employed in step (a) corresponds to a protonation degree of from 0.2 to 10%.

4. Process according to claim 3, characterised in that the amount of acid employed in step (a) corresponds to a protonation degree of from 0.3 to 7%.

5. Process according to claim 3, characterised in that the total amount of acid in step (b) corresponds to a protonation degree of from 20 to 50%.

6. Process according to claim 1, characterised in that the molar ratio of aromatic amine to formaldehyde is from 2:1 to 15:1.

7. Process according to claim 6, characterised in that the molar ratio of aromatic amine to formaldehyde is from 3:1 to 10:1.

8. Process according to claim 1, characterised in that hydrochloric acid is used as the acid catalyst.

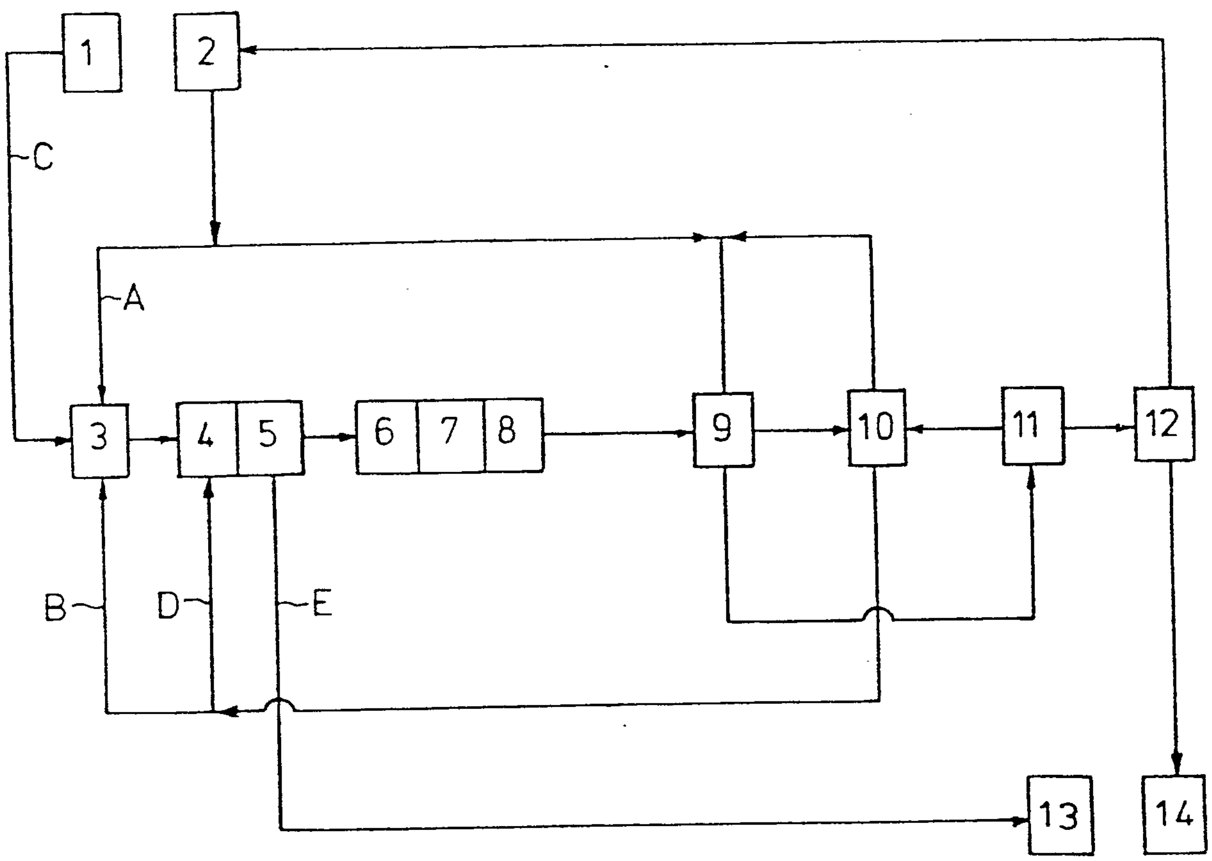
1
2
C
A
3
4
5
6
7
8
9
10
11
12
B
D
E
13
14